(19) Europäisches Patentamt
European Patent Office
Office européen des brevets

(11) Veröffentlichungsnummer: **0 059 365**
**A2**

(12) # EUROPÄISCHE PATENTANMELDUNG

(21) Anmeldenummer: 82101152.5

(22) Anmeldetag: 09.08.80

(51) Int. Cl.³: C 07 C 43/225
C 07 C 43/174, C 07 C 25/13
C 07 C 33/46, C 07 C 41/16
C 07 C 17/16, C 07 C 29/136

(30) Priorität: 22.08.79 DE 2933985

(43) Veröffentlichungstag der Anmeldung:
08.09.82 Patentblatt 82/36

(84) Benannte Vertragsstaaten:
BE CH DE FR GB IT LI NL

(60) Veröffentlichungsnummer der früheren Anmeldung nach Art. 76 EPÜ: 0 024 612

(71) Anmelder: BAYER AG
Zentralbereich Patente, Marken und Lizenzen
D-5090 Leverkusen 1, Bayerwerk(DE)

(72) Erfinder: Fuchs, Rainer, Dr.
Roeberstrasse 8
D-5600 Wuppertal 1(DE)

(72) Erfinder: Maurer, Fritz, Dr.
Roeberstrasse 8
D-5600 Wuppertal 1(DE)

(72) Erfinder: Priesnitz, Uwe, Dr.
Severingstrasse 58
D-5650 Solingen 1(DE)

(72) Erfinder: Riebel, Hans-Jochem, Dr.
In der Beek 92
D-5600 Wuppertal 1(DE)

(72) Erfinder: Klauke, Erich, Dr.
Eichendorffweg 8
D-5068 Odenthal(DE)

(54) **Neue 3-Brom-4-fluor-benzyl-Verbindungen und Verfahren zu ihrer Herstellung.**

(57) Die vorliegende Erfindung betrifft neue 3-Brom-4-fluor-benzyl-Verbindungen der Formel I

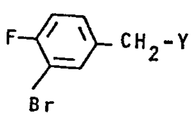

(I)

in welcher

Y für Phenoxy, Benzyloxy, Chlor, Brom oder OH steht, und ein Verfahren zu ihrer Herstellung, indem man für den Fall

Y = Phenoxy 3-Brom-4-fluor-benzylhalogenide mit Alkali- oder Erdalkali-phenolaten umsetzt und für den Fall Y = Benzyloxy 3-Brom-4-fluor-benzylalkohol mit Benzylhalogeniden umsetzt und für den Fall Y = Hal 3-Brom-4-fluorbenzoylalkohol mit Halogenierungsmitteln und für den Fall Y = OH 3-Brom-4-fluorbenzoyl-fluorid mit einem Hydridkomplex umsetzt.

EP 0 059 365 A2

Teilanmeldung aus der Europ. Anmeldung Nr. 80 104 715.0

BAYER AKTIENGESELLSCHAFT 5090 Leverkusen, Bayerwerk
Zentralbereich
Patente, Marken und Lizenzen Rt/bo
Typ IVb/ZP

# Neue 3-Brom-4-fluor-benzyl-Verbindungen und Verfahren zu ihrer Herstellung

Die Erfindung betrifft neue 3-Brom-fluor-benzyl-Verbindungen und Verfahren zu ihrer Herstellung.

Es ist bekannt, daß man 4-Fluor-3-phenoxy-benzyl-bromid, ein Zwischenprodukt für pestizid wirksame Pyrethroide, erhält, wenn man 4-Fluor-3-phenoxy-toluol mit N-Bromsuccinimid in Tetrachlorkohlenstoff unter Verwendung von Azodiisobuttersäurenitril als Katalysator umsetzt (vergleiche DE-OS 2 7o9 264). Diese Synthesemethode ist jedoch wegen der hohen Kosten der Reagentien und wegen der unbefriedigenden Ausbeuten für die Herstellung von 4-Fluor-3-phenoxy-benzyl-bromid im industriellen Maßstab wenig geeignet. 4-Fluor-3-phenoxybenzyl-bromid erhält man einfach und preiswert durch Etherspaltung von 4-Fluor-3-phenoxybenzylether mit Bromwasserstoff. 4-Fluor-3-phenoxybenzylether sind über die erfindungsgemäßen 3-Brom-4-fluorbenzyl-Verbindungen leicht zugänglich.

Die vorliegende Erfindung betrifft:

(1) neue 3-Brom-4-fluor-benzyl-Verbindungen der Formel I

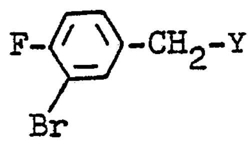

(I)

in welcher

Y für Phenyl, Benzyl, Chlor, Brom oder OH steht;

(2) ein Verfahren zur Herstellung von

(a) (3-Brom-4-fluor-benzyl)-phenyl-ether (Y = Phenyl), dadurch gekennzeichnet, daß man 3-Brom-4-fluor-benzylhalogenide der Formel II

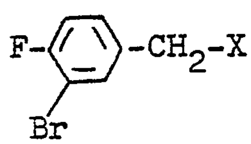

(II)

in welcher

X für Chlor oder Brom steht,

mit Alkali- oder Erdalkali-phenolaten gegebenenfalls in Gegenwart von Verdünnungsmitteln bei Temperaturen zwischen 0 und 150°C umsetzt;

(b) ein Verfahren zur Herstellung von (3-Brom-4-fluor-benzyl)-benzyl-ether (Y = Benzyl), dadurch gekennzeichnet, daß man 3-Brom-4-fluor-benzylalkohol der Formel III

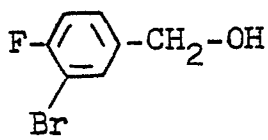

(III)

mit Benzylhalogeniden in Gegenwart von Basen und gegebenenfalls unter Verwendung von Verdünnungsmitteln bei Temperaturen zwischen 0 und 150°C umsetzt;

(c) ein Verfahren zur Herstellung der neuen 3-Brom-4-fluor-benzylhalogenide der Formel (II), dadurch gekennzeichnet, daß man 3-Brom-4-fluor-benzylalkohol der Formel III (oben) mit Halogenierungsmitteln, gegebenenfalls unter Verwendung von Verdünnungsmitteln, bei Temperaturen zwischen -10 und 100°C umsetzt;

(d) ein Verfahren zur Herstellung von 3-Brom-4-fluor-benzylalkohol der Formel III (oben), dadurch gekennzeichnet, daß man 3-Brom-4-fluor-benzoyl-fluorid der Formel IV

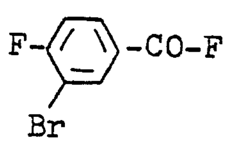

(IV)

mit einem Hydridkomplex der Formel V

$$M(M'H_4) \qquad (V)$$

in welchem

M für Lithium, Natrium oder Kalium steht und
M' für Bor oder Aluminium steht,

gegebenenfalls in Gegenwart eines Verdünnungsmittels bei Temperaturen zwischen 0 und 50°C umsetzt.

Mit Hilfe der neuen 3-Brom-4-fluor-benzyl-Verbindungen der Formel (I) kann 4-Fluor-3-phenoxy-benzylbromid wesentlich einfacher als nach dem oben erwähnten bekannten Verfahren hergestellt werden.

Dazu werden zunächst die 4-Fluor-3-phenoxy-benzylether z.B. nach folgendem Reaktionsschema hergestellt:

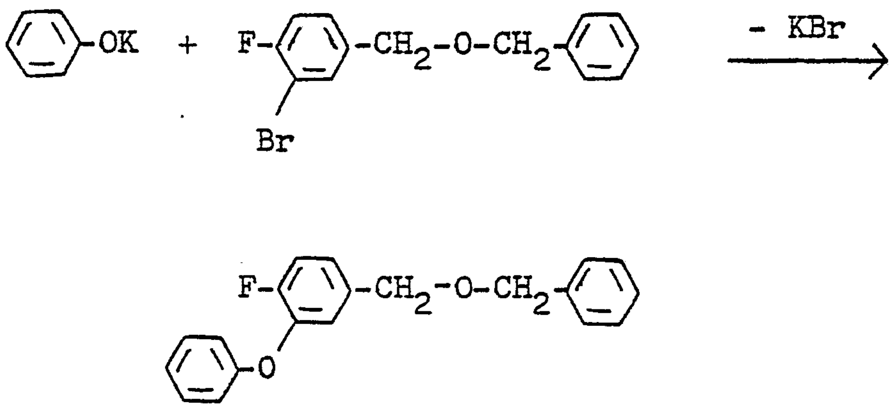


Alkali- oder Erdalkali-phenolate, welche bei diesem Verfahren als Ausgangsstoffe verwendet werden können, sind z.B. Natrium-, Kalium- und Magnesium-phenolat. Natriumphenolat wird als Ausgangsverbindung bevorzugt. Hilfsstoffe aus der Reihe der Alkali- oder Erdalkalihalogenide oder -carbonate sind z.B. Kalium- und Magnesium-chlorid sowie Kalium- und Magnesium-carbonat. Diese Hilfsstoffe werden vorzugsweise dann verwendet, wenn als Ausgangsverbindung Natriumphenolat eingesetzt wird.

Als Katalysatoren werden Kupfer oder Kupferverbindungen verwendet. Als Beispiele hierfür seien Kupfer, Kupfer(I)-oxid, Kupfer(II)oxid, Kupfer(I)chlorid und Kupfer(I)-bromid genannt.

Die Reaktionstemperatur wird bei Verfahren (2) zwischen 1oo und 2oo°C, vorzugsweise zwischen 14o und 18o°C gehalten. Das Verfahren wird gewöhnlich unter Normaldruck durchgeführt.

Auf 1 Mol 3-Brom-4-fluor-benzyl-ether der Formel (II) setzt man 1 bis 1,5 Mol, vorzugsweise 1 bis 1,2 Mol Phenolat, gegebenenfalls 1o bis 2oo g eines Hilfsstoffes aus der Reihe der Alkali- oder Erdalkali-halogenide oder -carbonate sowie o,o1 bis o,5 Mol, vorzugsweise o,1 bis o,5 Mol Kupferkatalysator ein.

In einer bevorzugten Variante (a) des Verfahrens zur Herstellung von (4-Fluor-3-phenoxy-benzyl)-benzyl-ether wird Isochinolin als Verdünnungsmittel verwendet. Zur Durchführung dieser Verfahrensvariante (a) werden die Komponenten vermischt und unter Rühren erhitzt, bis die Reaktion abgelaufen ist. Die Aufarbeitung kann auf übliche Weise durchgeführt werden. Beispielsweise wird das Reaktionsgemisch nach Abkühlen mit einem mit Wasser nicht

mischbaren Lösungsmittel, wie z.B. Cyclohexan, verdünnt, filtriert, das Filtrat mit Salzsäure und Wasser gewaschen, getrocknet, filtriert und das Lösungsmittel abdestilliert. Das zurückbleibende Rohprodukt kann durch Vakuumdestillation gereinigt werden.

In einer weiteren bevorzugten Variante (b) des Verfahrens zur Herstellung von (4-Fluor-3-phenoxy-benzyl)-phenylether wird Bis-(2-methoxyethyl)-ether (Diglyme) als Verdünnungsmittel verwendet. Zur Durchführung dieser Verfahrensvariante (b) werden die Komponenten vermischt und unter Rühren erhitzt, bis die Reaktion abgelaufen ist. Die Aufarbeitung kann auf übliche Weise durchgeführt werden. Beispielsweise wird das Reaktionsgemisch nach Abkühlen mit einem mit Wasser nicht mischbaren Lösungsmittel, wie z.B. Toluol, verdünnt und filtriert, das Filtrat mit verdünnter Natronlauge und mit Wasser gewaschen, getrocknet, filtriert und das Lösungsmittel abdestilliert. Das zurückbleibende Rohprodukt kann durch Vakuumdestillation gereinigt werden.

Die 4-Fluor-3-phenoxy-benzylether werden dann zur Herstellung von 4-Fluor-3-phenoxy-benzyl-bromid durch Etherspaltung mit Bromwasserstoffsäure eingesetzt. Dies kann bei Einsatz von (4-Fluor-3-phenoxy-benzyl)-benzyl-ether durch folgendes Formelschema skizziert werden:

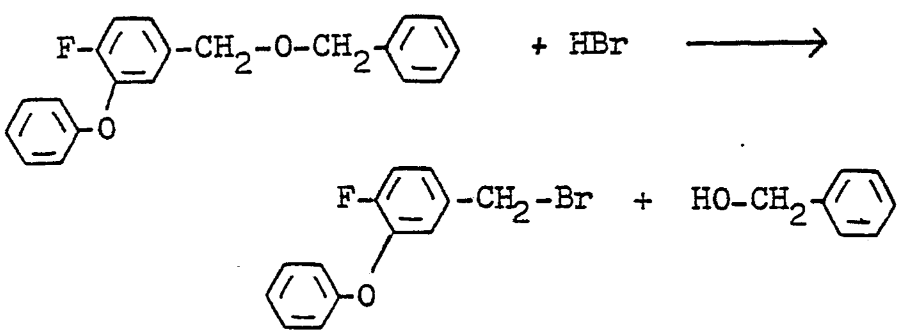

Die Etherspaltung zur Herstellung von 4-Fluor-3-phenoxy-benzylbromid kann nach üblichen Methoden durchgeführt werden.

In einer bevorzugten Verfahrensweise werden die Ether mit wäßriger Bromwasserstoffsäure und Essigsäure mehrere Stunden unter Rückfluß erhitzt. Zur Aufarbeitung wird das Reaktionsgemisch gegebenenfalls mit Wasser verdünnt und mit einem mit Wasser nicht mischbaren Lösungsmittel, wie z.B. Methylenchlorid, extrahiert. Die Extrakte werden mit verdünnter Natronlauge gewaschen und getrocknet. Dann wird das Lösungsmittel unter vermindertem Druck sorgfältig abdestilliert und das als Rückstand verbleibende Rohprodukt gegebenenfalls durch Vakuumdestillation gereinigt.

Das unter 2a (oben) dargelegte Verfahren zur Herstellung von (3-Brom-4-fluor-benzyl)-phenyl-ether ("Verfahren (2a)") kann bei Verwendung von 3-Brom-4-fluor-benzyl-chlorid und Kaliumphenolat als Ausgangsstoffen durch folgendes Formelschema skizziert werden:

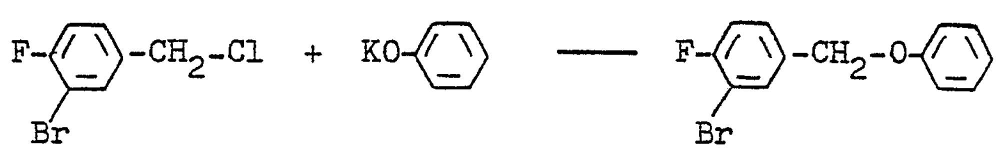

Alkali- oder Erdalkali-phenolate, welche bei Verfahren (2a) als Ausgangsstoffe verwendet werden können, sind z.B. Natrium-, Kalium- und Magnesium-phenolat. Natriumphenolat wird als Ausgangsverbindung bevorzugt.

Verfahren (2a) wird vorzugsweise unter Verwendung von Verdünnungsmitteln durchgeführt. Als solche kommen insbesondere aprotisch polare Lösungsmittel, wie z.B. Tetrahydrofuran, Glycoldimethylether, Acetonitril, Dimethylformamid oder Dimethylsulfoxid in Frage.

Die Reaktionstemperatur wird bei Verfahren (2a) zwischen 0 und 15o°C, vorzugsweise zwischen lo und loo°C gehalten. Das Verfahren wird im allgemeinen bei Normaldruck durchgeführt.

Auf 1 Mol 3-Brom-4-fluor-benzyl-halogenid (II) setzt man 1 bis 1,5 Mol, vorzugsweise 1 bis 1,2 Mol Phenolat ein. In einer bevorzugten Ausführungsform von Verfahren (2a) wird das Phenolat in einem Verdünnungsmittel vorgelegt und dazu unter Rühren tropfenweise das 3-Brom-4-fluor-benzyl-halogenid gegeben. Das Reaktionsgemisch wird dann, gegebenenfalls bei mäßig erhöhter Temperatur bis zum Reaktionsende gerührt. Die Aufarbeitung kann auf übliche Weise durchgeführt werden, beispielsweise indem man mit Wasser verdünnt und mit einem mit Wasser nicht mischbaren Lösungsmittel, wie z.B. Methylenchlorid, extrahiert, die Extrakte trocknet, filtriert und einengt und den Rückstand gegebenenfalls im Vakuum destilliert.

Das unter (2b) dargelegte Verfahren zur Herstellung von (3-Brom-4-fluor-benzyl)-benzyl-ether ("Verfahren (2b)") kann bei Verwendung von Benzylbromid als Reaktionskomponente durch folgendes Formelschema skizziert werden:

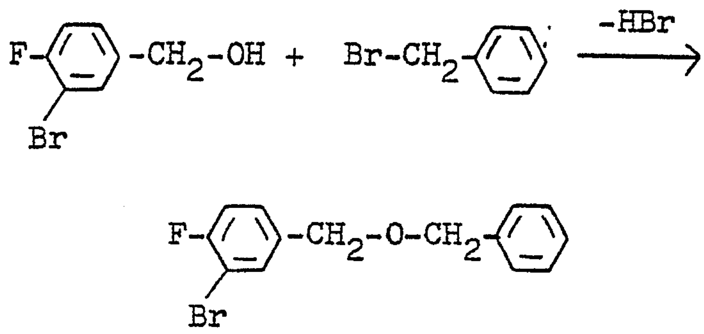


Verfahren (2b) wird vorzugsweise unter Verwendung von Verdünnungsmitteln durchgeführt. Es werden die gleichen Lösungsmittel wie in Verfahren (2a) bevorzugt.

Benzylhalogenide, welche bei Verfahren (2b) bevorzugt eingesetzt werden, sind Benzylchlorid und Benzylbromid.

Bei Verfahren (2b) werden zur Deprotonierung von Benzylalkoholen geeignete Basen verwendet. Als solche kommen Alkoholate, wie z.B. Natriummethylat und Kalium-tert.-butylat, Alkaliamide, wie z.B. Natriumamid und Alkalihydride, wie z.B. Natriumhydrid, in Frage.

Die Reaktionstemperatur wird bei Verfahren (2b) zwischen 0 und 15o°C, vorzugsweise zwischen lo und loo°C gehalten. Das Verfahren wird im allgemeinen bei Normaldruck durchgeführt.

Auf 1 Mol 3-Brom-4-fluor-benzylalkohol setzt man 1 bis 1,5 Mol, vorzugsweise 1 bis 1,2 Mol Base und 1 bis 1,5 Mol, vorzugsweise 1 bis 1,2 Mol Benzylhalogenid ein. In einer bevorzugten Ausführungsform von Verfahren (2b)

wird die Base in einem Verdünnungsmittel vorgelegt und 3-Brom-4-fluor-benzylalkohol dazu gegeben. Nach etwa einer Stunde wird hierzu das Benzylhalogenid tropfenweise gegeben und das Gemisch wird bis zum Reaktionsende, gegebenenfalls bei mäßig erhöhter Temperatur, gerührt. Die Aufarbeitung kann auf übliche Weise durchgeführt werden, beispielsweise indem man das Reaktionsgemisch mit verdünnter Salzsäure versetzt, mit einem mit Wasser nicht mischbaren Lösungsmittel, wie z.B. Diethylether extrahiert, den Etherextrakt trocknet, filtriert und destilliert.

Das Verfahren zur Herstellung von 3-Brom-4-fluor-benzylhalogeniden aus 3-Brom-4-fluor-benzyl-alkohol ("Verfahren (2c)") wird vorzugsweise unter Verwendung von Verdünnungsmitteln durchgeführt. Als solche kommen vor allem aliphatische und aromatische, gegebenenfalls halogenierte Kohlenwasserstoffe, wie z.B. Pentan, Hexan, Heptan, Cyclohexan, Petrolether, Ligroin, Benzol, Toluol, Xylol, Methylenchlorid, Ethylenchlorid, Chloroform, Tetrachlorkohlenstoff, Chlorbenzol und o-Dichlorbenzol in Frage.

Bei Verfahren (2c) zu verwendende Halogenierungsmittel sind z.B. Thionylchlorid, Phosphortrichlorid, Phosphorpentachlorid, Phosphortribromid und Dibrom-triphenylphosphoran.

Die Reaktionstemperatur liegt bei Verfahren (2c) zwischen -lo und loo°C, vorzugsweise zwischen 0 und 5o°C. Das Verfahren wird im allgemeinen bei Normaldruck durchgeführt.

Zur Durchführung von Verfahren (2c) wird 3-Brom-4-fluorbenzylalkohol, gegebenenfalls in einem der oben angegebenen Verdünnungsmittel, vorgelegt und mit wenigstens der äquivalenten Menge eines Halogenierungsmittels tropfenweise versetzt. Nach Ende der Reaktion kann das Produkt durch Destillation in reiner Form isoliert werden. Eine andere Form der Aufarbeitung besteht darin, daß man das Reaktionsgemisch mit Wasser verdünnt, die organische Phase abtrennt, trocknet, filtriert und destilliert.

Das Verfahren zur Herstellung von 3-Brom-4-fluor-benzylalkohol ("Verfahren (2d)") wird vorzugsweise unter Verwendung von Verdünnungsmitteln durchgeführt. Als solche kommen vor allem Alkohole, wie z.B. Methanol, Ethanol, n- und iso-Propanol, n-, iso-, sek.- und tert.-Butanol in Frage. Iso-Propanol wird als Verdünnungsmittel besonders bevorzugt.

Bei Verfahren (2d) als Reduktionsmittel zu verwendende Hydridkomplexe der Formel (V) sind z. B. Lithium-tetrahydridoaluminat (Lithiumalanat) und Natriumtetrahydridoborat (Natriumboranat). Letzteres wird besonders bevorzugt.

Verfahren (2d) wird bei Temperaturen zwischen 0 und 5o°C, vorzugsweise zwischen lo und 4o°C, und gewöhnlich bei Normaldruck durchgeführt.

In einer bevorzugten Ausführungsform von Verfahren (2d) wird der Hydridkomplex der Formel (V) in einem der oben angegebenen Verdünnungsmittel vorgelegt und 3-Brom-4-fluor-benzoylfluorid langsam zugetropft. Das Reaktionsgemisch wird bis zum Ende der Umsetzung gerührt, mit Eiswasser verdünnt und angesäuert. Dann wird mit einem mit Wasser nicht mischbaren Lösungsmittel, wie z.B. mit Methylenchlorid, extrahiert, der Extrakt getrocknet, filtriert und destilliert.

Das als Ausgangsverbindung bei Verfahren (2d) zu verwendende 3-Brom-4-fluor-benzoylfluorid ist Gegenstand einer noch nicht zum Stand der Technik gekennzeichneten deutschen Patentanmeldung (vergleiche P 2 915 738 / Le A 19 590).

Man erhält diese Verbindung, wenn man 4-Chlor-benzoylchlorid durch Umsetzung mit Kaliumfluorid in 4-Fluorbenzoylfluorid umwandelt und letzteres zu 3-Brom-4-fluorbenzoylfluorid bromiert gemäß nachstehendem Formelschema:

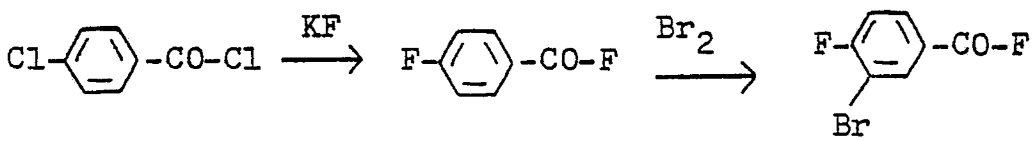


4-Chlor-benzoylchlorid wird mit Kaliumfluorid beispielsweise in Tetramethylensulfon bei Temperaturen zwischen 2oo und 22o°C umgesetzt und das Reaktionsgemisch destillativ aufgearbeitet. Man erhält 4-Fluor-benzoylfluorid vom Siedepunkt 53°C/2o mBar (Brechungsindex: $n_D^{2o}$=1,4792).

4-Fluor-benzoylfluorid wird mit elementarem Brom in Gegenwart von 1% Eisen(III)chlorid bei 7o bis 75°C umgesetzt. Bei einem Ansatz von 1 Mol erhält man nach Destillation 4o g unverändertes Ausgangsmaterial zurück

und 182 g eines Gemisches aus 3-Brom-4-fluor-benzoylfluorid (Siedepunkt: 82-83°C/15 mBar; Brechungsindex: $n_D^{20}$ = 1,5315; Schmelzpunkt: 32-34°C) und 3-Brom-4-fluor-benzoylbromid (Siedepunkt: 123°C/15 mBar; Schmelzpunkt: 35-37°C).

Beispiel 1 (nicht erfindungsgemäß)

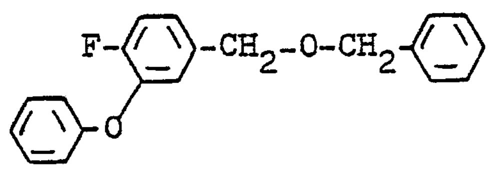

Eine Mischung von 1 Mol (3-Brom-4-fluor-benzyl)-benzyl-ether, 1,1 Mol Natriumphenolat, o,12 Mol Kupfer(I)oxid, 8o g 4 $MgCO_3 \cdot Mg(OH)_2 \cdot 4\ H_2O$ und 15oo ml Isochinolin wird 12 Stunden auf 16o°C erhitzt. Dann wird das Reaktionsgemisch abgekühlt, mit Cyclohexan verdünnt und filtriert. Das Filtrat wird mit verdünnter Salzsäure und Wasser gewaschen, über Natriumsulfat getrocknet, filtriert und destilliert. Man erhält (4-Fluor-3-phenoxy-benzyl)-benzyl-ether in einer Ausbeute von 75% der Theorie in Form eines gelben Öls vom Siedepunkt 135°C/ 1 mBar.

Ersetzt man in Beispiel 1 das Magnesiumcarbonat durch 14o g Kaliumcarbonat und verwendet o,2 Mol Kupfer(I)-oxid als Katalysator, so erhält man bei einer Reaktionszeit von 8 Stunden das gleiche Produkt in einer Ausbeute von 7o% der Theorie.

Beispiel 2 (nicht erfindungsgemäß)

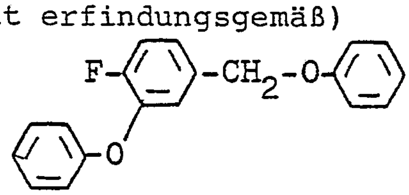

Eine Mischung von 84,3 g (o,3 Mol) (3-Brom-4-fluor-benzyl)-phenyl-ether, 34,8 g (o,3 Mol) Natriumphenolat,

7 g (o,11 Mol) Kupferpulver, lo g Kaliumchlorid und 5o ml Bis-(2-methoxyethyl)-ether (Diglyme) wird unter Rühren 5 Stunden lang auf 16o°C erhitzt. Der Reaktionsansatz wird dann auf 8o°C abgekühlt, mit 3oo ml Toluol versetzt und filtriert. Das Filtrat wird 1 mal mit loo ml lo%igem Natriumsulfat gewaschen, anschließend 2 mal mit je 3oo ml Wasser ausgeschüttelt. Die organische Phase wird über Magnesiumsulfat getrocknet und das Lösungsmittel im Wasserstrahlvakuum abgezogen. Der ölige Rückstand wird im Vakuum destilliert. Man erhält 7o,7 g (8o,2% der Theorie)(4-Fluor-3-phenoxy-benzyl)-phenylether mit dem Siedepunkt: 195-197°C/3 mBar als sehr zähes Öl.

Beispiel 3 (erfindungsgemäß)

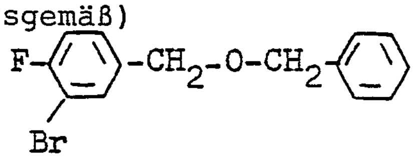

Zu einer Lösung von 12,3 g (o,11 Mol) Kalium-tert.-butylat in 2oo ml Tetrahydrofuran gibt man bei 2o°C 2o,5 g (o,1 Mol) 3-Brom-4-fluor-benzylalkohol. Dabei steigt die Temperatur auf ca. 4o°C. Man läßt ca. 1 Stunde bei 2o°C nachrühren und tropft dann 12,6 g (o,1 Mol) Benzylchlorid zum Reaktionsgemisch. Dann wird eine Stunde unter Rückfluß erhitzt. Anschließend wird abgekühlt. Es werden ca. 2oo ml Wasser und 5o ml konzentrierter Salzsäure zum Reaktionsgemisch gegeben, und es wird zweimal mit je 2oo ml Diethylether extrahiert. Die vereinigten Extrakte werden eingeengt, über Natriumsulfat getrocknet und dann fraktioniert destilliert. Man erhält 14,2 g (48,2% der Theorie) (3-Brom-4-fluor-benzyl)-benzyl-ether in Form eines hellgelben Öls vom Siedepunkt 165°C/7 mBar.

Beispiel 4 (erfindungsgemäß)

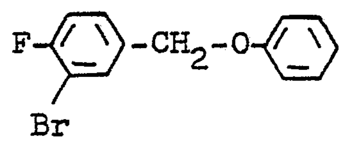

Zu einer Suspension von 5,8 g (o,o5 Mol) Natriumphenolat in loo ml Acetonitril werden bei 2o-25°C unter Rühren 13,4 g (o,o5 Mol) 3-Brom-4-fluor-benzylbromid zugetropft. Anschließend wird unter Rühren 3 Stunden lang auf 8o°C erhitzt. Nach dem Abkühlen wird der Reaktionsansatz in 5ooml Wasser gegossen und 2 mal mit je 2oo ml Methylenchlorid extrahiert. Die organische Phase wird abgetrennt, über Magnesiumsulfat getrocknet und das Lösungsmittel im Wasserstrahlvakuum abgezogen. Der Rückstand wird im Vakuum destilliert, man erhält 12,6 g (9o% der Theorie) (3-Brom-4-fluor-benzyl)-phenyl-ether mit dem Siedepunkt 145-147°C/3 mBar.

Beispiel 5 (erfindungsgemäß)

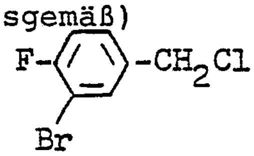

41 g (o,2 Mol) 3-Brom-4-fluor-benzylalkohol werden in 25o ml Tetrachlorkohlenstoff gelöst und bei 2o°C unter Rühren 26 g Thionylchlorid zugetropft. Dann wird 4 Stunden bei 25-3o°C gerührt, anschließend das Lösungsmittel und überschüssiges Thionylchlorid im Wasserstrahlvakuum bei 2o°C abgezogen. Der verbleibende Rückstand wird im Vakuum destilliert. Man erhält 29 g (64,9% der Theorie) 3-Brom-4-fluor-benzylchlorid mit dem Siedepunkt 85-87°C/ 3 mBar.

Beispiel 6 (erfindungsgemäß)

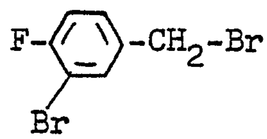

2o,5 g (o,1 Mol) 3-Brom-4-fluor-benzylalkohol werden in 1oo ml wasserfreiem Toluol gelöst und bei 0 bis 1o°C unter Rühren 1o g Phosphortribromid zugetropft. Dann wird 2 Stunden bei Raumtemperatur gerührt. Anschließend wird der Reaktionsansatz in 5oo ml Wasser gegossen, die organische Phase wird abgetrennt, über Magnesiumsulfat getrocknet und das Lösungsmittel im Wasserstrahlvakuum abdestilliert. Der Rückstand wird im Vakuum destilliert. Man erhält 24 g (89,6% der Theorie) 3-Brom-4-fluor-benzylbromid mit dem Siedepunkt 99-1oo°C/3 mBar.

Beispiel 7 (erfindungsgemäß)

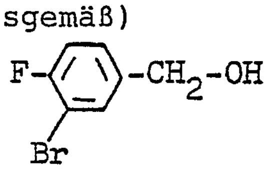

221 g (1 Mol) 3-Brom-4-fluor-benzoylfluorid werden zu einer Mischung von 3o,4 g (o,8 Mol) Natriumtetrahydridoborat und 84o ml iso-Propanol bei 2o-25°C innerhalb von 3 Stunden tropfenweise gegeben. Das Reaktionsgemisch wird noch ca. 3o Minuten gerührt und dann in 2 l Eiswasser gegossen. Durch Zugabe von konzentrierter Salzsäure wird pH 1 eingestellt und dann die organische Schicht abgetrennt. Die wässrige Phase wird mit 2oo ml Methylenchlorid nachextrahiert. Die vereinigten organischen Phasen werden getrocknet, filtriert und destilliert. Man erhält 163 g (79,5% der Theorie) 3-Brom-4-fluor-benzylalkohol vom Siedepunkt 137°C/16 mBar und vom Brechungsindex $n_D^{20}$ = 1,5623.

Beispiel zur Herstellung von 4-Fluor-3-phenoxy-benzylbromid:

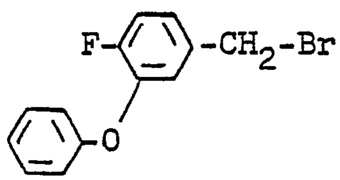

Eine Mischung von 14,7 g (o,o5 Mol) (4-Fluor-3-phenoxy-benzyl)-phenyl-ether, 5o ml 32%ige wässrige Bromwasserstoffsäure und 6o ml Eisessig wird unter Rühren lo Stunden zum Rückfluß erhitzt. Anschließend wird die Reaktionsmischung in 2oo ml Wasser gegossen und 2 mal mit je 2oo ml Methylenchlorid extrahiert. Die vereinigten Methylenchloridphasen werden anschließend 2 mal mit je looml lo%iger Natronlauge ausgeschüttelt. Anschließend wird die organische Phase über Magnesiumsulfat getrocknet und dann das Lösungsmittel im Wasserstrahlvakuum abgezogen. Der verbleibende Rückstand wird durch Andestillieren bei 6o°C Badtemperatur/3 mBar von den letzten Lösungsmittelresten befreit. Man erhält so das 4-Fluor-3-phenoxy-benzylbromid als zähes Öl.

Patentansprüche

1. 3-Brom-4-fluor-benzyl-Verbindungen der Formel I

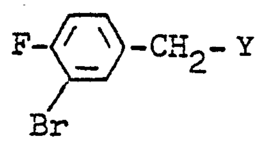

(I)

in welcher

Y für Phenyl, Benzyl, Chlor, Brom oder OH steht.

2. a) Verfahren zur Herstellung von (3-Brom-4-fluor-benzyl)-phenyl-ether der Formel I, in welcher Y für Phenyl steht, dadurch gekennzeichnet, daß man 3-Brom-4-fluor-benzylhalogenide der Formel II

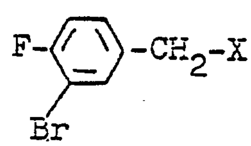

(II)

in welcher

X für Chlor oder Brom steht,

mit Alkali- oder Erdalkali-phenolaten gegebenenfalls in Gegenwart von Verdünnungsmitteln bei Temperaturen zwischen 0 und 150°C umsetzt;

b) Verfahren zur Herstellung von (3-Brom-4-fluor-benzyl)-benzyl-ether der Formel I, in welcher Y für Benzyl steht, dadurch gekennzeichnet, daß man 3-Brom-4-fluor-benzylalkohol der Formel III

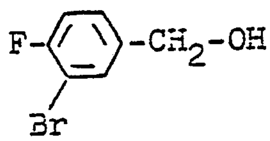

(III)

mit Benzylhalogeniden in Gegenwart von Basen und gegebenenfalls unter Verwendung von Verdünnungsmitteln bei Temperaturen zwischen 0 und 150°C umsetzt;

c) Verfahren zur Herstellung der 3-Brom-4-fluor-benzylhalogenide der Formel (II), dadurch gekennzeichnet, daß man 3-Brom-4-fluor-benzylalkohol der Formel III mit Halogenierungsmitteln, gegebenenfalls unter Verwendung von Verdünnungsmitteln, bei Temperaturen zwischen -10 und 100°C umsetzt;

d) Verfahren zur Herstellung von 3-Brom-4-fluor-benzylalkohol der Formel III, dadurch gekennzeichnet, daß man 3-Brom-4-fluor-benzoylfluorid der Formel IV

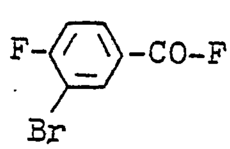

(IV)

mit einem Hydridkomplex der Formel V

$$M(M'H_4) \qquad (V)$$

in welchem

M für Lithium, Natrium oder Kalium steht und
M' für Bor oder Aluminium steht,

gegebenenfalls in Gegenwart eines Verdünnungsmittels bei Temperaturen zwischen 0 und 50°C umsetzt.